# EUROPÄISCHE PATENTSCHRIFT

(11) **EP 1 967 221 B1**
(45) Veröffentlichungstag und Bekanntmachung des Hinweises auf die Patenterteilung: **13.08.2014**
(21) Anmeldenummer: 08011526.4
(22) Anmeldetag: 31.03.2003
(51) Int. Cl.: A61M 1/16, A61M 1/28, A61K 31/715

(54) **Verfahren zur Bereitstellung einer Lösung für die Peritonealdialyse**
Method of providing a solution for peritoneal dialysis
Méthode pour la préparation d'une solution pour la dialyse péritoneale

(30) Priorität: 18.04.2002 DE 10217356
(43) Veröffentlichungstag der Anmeldung: 10.09.2008
(62) Teilanmeldung aus: 05025667.6
(73) Patentinhaber: Fresenius Medical Care Deutschland GmbH, 61352 Bad Homburg v.d.H. (DE)
(72) Erfinder: Zimmeck, Thomas Dr., D-25551 Hohenlockstedt (DE)
(74) Vertreter: Herrmann, Uwe

(56) Entgegenhaltungen:
- EP-A- 0 170 275
- EP-A- 0 564 672
- EP-A- 0 602 585
- WO-A-95/19778
- DE-A- 19 748 290

## Beschreibung

Die vorliegende Erfindung betrifft ein Verfahren zur Herstellung einer Lösung für die Peritonealdialyse gemäß dem Oberbegriff des Anspruchs 1.

Lösungen für die Peritonealdialyse enthalten im Wesentlichen drei Komponenten: das Puffersystem, Elektrolyte und ein Osmotikum. Als Osmotikum, das in osmotisch wirksamer Konzentration im Wesentlichen dazu dient, den Wassergehalt des Blutes zu verringern, wird häufig Glucose verwendet, die eine gute Osmolarität aufweist und zudem gut verträglich ist. Ein weiterer Vorteil des Einsatzes von Glucose ist der Preisvorteil gegenüber anderen in Frage kommenden Osmotika.

Ein Nachteil bei der Verwendung von Glucose besteht allerdings darin, daß diese bei der Hitzesterilisation karamelisiert, isomerisiert oder daß Abbauprodukte gebildet werden, die im Körper des Patienten schädliche Wirkungen entfalten, beispielsweise mit Proteinen weiterreagieren, was unerwünscht ist. Um diesen Nachteilen vorzubeugen, ist es aus der DE 197 48 290 A1 bekannt, eine aus zwei Einzellösungen bestehende Peritonealdialyselösung einzusetzen, wobei in der die Glucose sowie Elektrolytsalze enthaltenden Einzellösung ein pH-Wert von unter 3,2 eingestellt wird. Um bei der Mischung der Einzellösungen einen physiologisch verträglichen pH-Wert zu erhalten, wird ferner offenbart, in einer zweiten alkalischen Einzellösung außer dem in geringer Konzentration vorliegenden Bicarbonat das Salz einer schwachen Säure mit pKa < 5 vorzulegen. Diese beiden Einzellösungen werden nach der Hitzesterilisation miteinander gemischt und die Mischung wird sodann dem Patienten zugeführt. Bei geringen pH-Werten von unter 3,2 kann der Abbau von Glucose weitgehend verhindert werden.

Neben der genannten Verwendung von Glucose als Osmotikum ist es beispielsweise aus der WO 83/00087 bekannt, Glucosepolymere als Ersatz oder zusätzlich zu Glucose einzusetzen. Glucosepolymere werden insbesondere für lange Verweilzeiten in Peritonealdialyselösungen eingesetzt, weil sie ein günstiges Ultrafiltrationsprofil aufweisen. Aufgrund der im Gegensatz zu Glucose verhältnismäßig langsamen Diffusionsgeschwindigkeit von Glucosepolymeren bleibt die Osmolarität während der Behandlung im Wesentlichen erhalten. Zudem wird die Belastung der Patienten mit Glucose verringert, was insbesondere bei Diabetes-Patienten von Vorteil ist.

Der bei der Verwendung von Glucose beobachtete Abbau bei annähernd neutralen pH-Werten, insbesondere in Gegenwart von Lactat, und die Umwandlung, beispielsweise zu Fructose, Acetaldehyd und 3-Desoxyglucoson gilt in eingeschränktem Umfang auch für Glucosepolymere und Glucosepolymer-Derivate. Aus diesem Grund können Glucosepolymere bzw. Glucosepolymer-Derivat enthaltende Lösungen nicht bei neutralen pH-Werten sterilisiert werden.

Die nachfolgende Abbildung zeigt die Konzentration des Abbauproduktes 3-Desoxyglucoson für unterschiedliche das Osmotikum enthaltende, in Doppelkammerbeuteln befindliche Einzellösungen, wobei die rechts dargestellte Lösung statt Glucose ein Glucosepolymer als Osmotikum enthält. Daraus wird ersichtlich, daß auch bei der Verwendung von Glucosepolymeren verhältnismäßig große Mengen an Abbauprodukten gefunden werden. Dies ist darauf zurückzuführen, daß nicht nur die endständige Carbonylgruppe von Glucosepolymeren umgewandelt wird, sondern sich auch eine Glucoseeinheit vom Polymer abspalten kann. Darüber hinaus ist mit bislang unbekannten Umwandlungsprodukten zu rechnen, die sich noch im Polymerverbund des Osmotikums befinden.

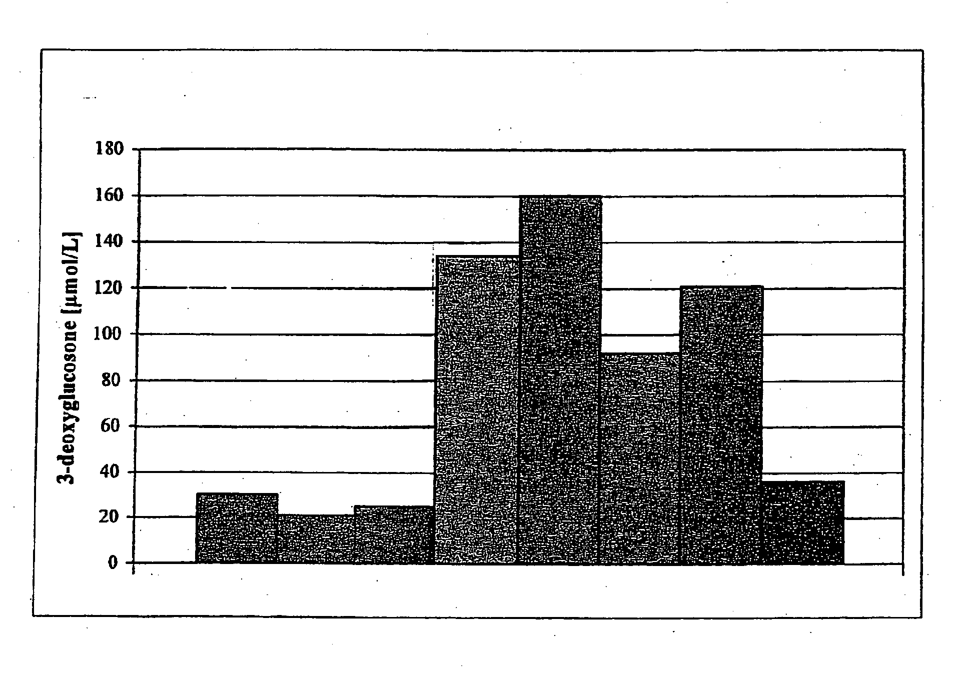

In der bereits genannten WO 83/00087 werden Peritonealdialyselösungen beschrieben, bei denen als Osmotikum Glucosepolymere mit einem Polymerisationsgrad von wenigstens 4 eingesetzt werden. Die Peritonealdialyselösung dieser Druckschrift weist einen pH-Bereich von 5 bis 7,4 auf, was bei der Hitzesterilisation mit den oben genannten Nachteilen verbunden ist.

Will man die Probleme hinsichtlich des Abbaus bzw. der Umwandlung von Glucosepolymeren oder deren Derivate bei der Lagerung und Hitzesterilisation vermeiden, indem man den pH-Wert auf Werte von unter 3,2 einstellt, wie dies für den Fall von Glucose aus der DE 197 48 290 A1 bekannt ist, ergibt sich das Problem, daß die Polymere hydrolysiert werden, was in einem Bruch der Polymerkette bzw. in der Verringerung des mittleren Molekulargewichts resultiert. Erschwert wird die Herstellung von Glucosepolymer bzw. Glucosepolymerderivat haltigen Lösungen dadurch, daß diese möglicherweise Säuren enthalten, was bei der pH-Wert-Einstellung zu berücksichtigen ist.

Die EP 0 564 672 offenbart des weiteren eine wäßrige Peritonealdialyselösung, die aus zwei Einzellösungen erhalten wird, wobei der pH-Wert der ersten Einzellösung im Bereich zwischen 4,5 und 5,8 liegt. Die EP 0 602 585 A2 betrifft Dialyselösungen für die Peritonealdialyse, die Hydroxyethylstärke als osmotisch wirksame Substanz enthalten.

Es ist daher die Aufgabe der vorliegenden Erfindung ein Verfahren zur Herstellung einer Peritonealdialyselösung zur Verfügung zu stellen, wobei in der Lösung enthaltene Glucosepolymere und/oder Glucosepolymer-Derivate bei der Lagerung und der Hitzesterilisation keinem glucoseähnlichen Abbau unterliegen und auch nicht hydrolysiert werden, und wobei die Lösung einen Misch-pH-Wert im neutralen Bereich aufweisen.

Diese Aufgabe wird erfindungsgemäß durch ein Verfahren gemäß Anspruch 1 gelöst. Besonders vorteilhaft ist es, wenn der pH-Wert der ersten Einzellösung bei 4,2 liegt. Bei diesen pH-Werten ist annähernd kein Polymerabbau zu beobachten. Dies gilt insbesondere für einen pH-Wert von 4,0. Die 0,2 pH-Einheiten zu dem Vorzugswert von 4,2 sind als Sicherheitszuschlag für die mögliche Bildung von Säuren während der Sterilisation und Lagerung bestimmt. In dem beanspruchten pH-Bereich findet in merklichem Umfang weder die Hydrolyse des Osmotikums noch dessen glucoseähnlicher Abbau statt. Das Osmotikum kann ausschließlich durch das Glucosepolymer und/oder das Glucosepolymer-Derivat gebildet werden. Denkbar ist auch, daß weitere osmotisch wirksame Substanzen enthalten sind.

In weiterer Ausgestaltung der vorliegenden Erfindung ist vorgesehen, daß es sich bei dem Glucosepolymer-Derivat um Hydroxyethylstärke (HES) handelt. Die vorliegende Erfindung bezieht sich auch auf andere derivatisierte Glucosepolymere, bei denen vorzugsweise bei der Derivatisierung nicht die freie Carbonylgruppe des Moleküls verändert wurde.

Die erste Einzellösung kann das Osmotikum, Calcium-lonen, Magnesium-lonen, Natrium-lonen, H⁺-Überschußionen und Chlorid-lonen enthalten.

In bevorzugter Ausgestaltung der vorliegenden Erfindung enthält der Puffer Bicarbonat. Es handelt sich dabei um ein sehr verträgliches Puffersystem, das im basischen Bereich mit Carbonat und im sauren Bereich mit CO₂ im Gleichgewicht steht. Außer oder neben Bicarbonat sind auch andere Puffersysteme denkbar, die in einem physiologischen pH-Wert von ca. 7 puffern. Hierbei sind vorzugsweise Stoffe zu nennen, die im Körper leicht zu Bicarbonat abgebaut werden können. In Betracht kommen beispielsweise Lactat oder Pyruvat. Neben Bicarbonat oder anderen Puffersystemen enthält die zweite Einzellösung meist ferner Natrium-Ionen.

Vorteilhaft ist es, wenn die Bicarbonatkonzentration in Abhängigkeit von der Acidität der ersten Einzellösung eingestellt wird und nach der Formel: Bicarbonatkonzentration [mmol/l] = 5 x Acidität der ersten Einzellösung [mmol/l] x V_{A}V_{B} bestimmt wird, wobei V_{A} das Volumen der ersten Einzellösung und V_{B} das Volumen der zweiten Einzellösung darstellt.

Bei einer Acidität von 0,2 mmol/l beträgt die optimale Bicarbonatkonzentration bei gleich großen Kompartimenten eines Doppelkammerbeutels 0,5 bis 2,0 mmol/l. Dementsprechend kann die Bicarbonatkonzentration in einem Bereich liegen, dessen Untergrenze durch die Hälfte der nach Anspruch 6 bestimmten und dessen Obergrenze durch das Doppelte der nach Anspruch 6 bestimmten Bicarbonatkonzentration gebildet wird.

In weiterer Ausgestaltung der vorliegenden Erfindung ist vorgesehen, daß der Puffer das Salz einer schwachen Säure, vorzugsweise Lactat, enthält. Der pKa-Wert der schwachen Säure kann bei < 5 liegen. Es kann vorgesehen sein, daß der Puffer eine Mischung, beispielsweise aus Bicarbonat und dem Salz einer schwachen Säure, beispielsweise Lactat, enthält. Wird der Gehalt von Bicarbonat gering gehalten, beispielsweise < 10 mmol/l, wie dies in der DE 197 48 290 A1 vorgeschlagen wird, hat dies den Vorteil, daß der Druck des CO₂ innerhalb des Aufbewahrungsbeutels gering ist, so daß keine besonderen Vorkehrungen hinsichtlich der Beutelfolie vorgesehen werden müssen. Als CO₂-Barriere kann hier eine übliche Polyolefinfolie eingesetzt werden.

Die erste Einzellösung kann eine physiologisch verträgliche Säure, insbesondere Salzsäure enthalten. Mit dieser läßt sich ohne weiteres der gewünschte pH-Wert-Bereich der ersten Einzellösung einstellen.

Die erste Einzellösung kann außer dem Osmotikum folgende Bestandteile aufweisen:
Natrium-Ionen [mmol/l]: 180 - 200
Calcium-Ionen [mmol/l]: 2 - 4
Magnesium-Ionen [mmol/l]: 0,8- 1,2
H⁺-Überschuß [mmol/l]: 0,05 - 0,1
Chlorid-Ionen [mmol/l]: 197 - 210

In weiterer Ausgestaltung der vorliegenden Erfindung ist vorgesehen, daß die Bicarbonatkonzentration der zweiten Einzellösung im Bereich zwischen 0,5 und 2,0 mmol/l, vorzugsweise bei 1,0 mmol/l liegt.

Gemäß der Erfindung werden die erste und zweite Einzellösung in einem Doppelkammerbeutel getrennt gelagert. Durch die Verwendung eines Doppelkammerbeutels ergibt sich ein besonders gutes Handling der Lösung, d. h. eine zuverlässige Trennung der beiden Einzellösungen während deren Lagerung und ein schnelles Vermischen im Bedarfsfall. Die Trennung der Einzellösungen ist sinnvoll, um zu vermeiden, daß sich bei der Verwendung von Bicarbonat als Puffer und Calcium unlösliche Niederschläge bilden. Darüber hinaus läßt sich die Reaktion der Glucosepolymere bzw. deren Derivate mit Lactat als Puffersystem durch die Trennung vermeiden.

Der Doppelkammerbeutel ist vorzugsweise ein Kunststoffbeutel. Er weist wenigstens eine erste und eine zweite Kammer auf, wobei die erste Einzellösung in der ersten Kammer und die zweite Einzellösung in der zweiten Kammer aufgenommen ist. Es sind Mittel vorgesehen, durch die beide Kammern voneinander getrennt sind und bei deren Betätigung der Inhalt beider Kammern miteinander vermischbar ist. Die erste und zweite Kammer sind benachbart angeordnet. Es ist eine Schweißnaht vorgesehen, die die Kammern voneinander trennt und die sich bei einem Druck auf eine der Kammern öffnet. Bei Druck auf eine der flüssigkeitsgefüllten Kammern öffnet sich die Schweißnaht, so daß der Inhalt der beiden Kammern miteinander vermischbar ist und die Mischung schließlich dem Patienten verabreicht werden kann.

Im Folgenden wird ein Beispiel für die erfindungsgemäße Herstellung einer Lösung gegeben:

Zur Herstellung der ersten Einzellösung werden in Wasser Natriumchlorid, Calciumchlorid, Magnesiumchlorid sowie ein Glucosepolymer und Salzsäure unter Rühren in Lösung gebracht. Die Menge der zugegebenen Salzsäure wird derart eingestellt, daß der pH-Wert im Bereich von 4,1 bis 4,3, vorzugsweise bei 4,2 liegt. Während ein pH-Wert von 4,0 als ideal anzusehen ist, da hier kein Polymerabbau zu beobachten ist, dienen die 0,2 pH-Einheiten zu einem pH-Wert von 4,2 als Zuschlag für die mögliche Bildung von Säuren während der Sterilisation und Lagerung.

Die Acidität dieser ersten Einzellösung kann durch Titration mit 0,1 N NaOH bis pH 7,0 bestimmt werden.

Bei der zweiten Einzellösung wird in Wasser Natriumhydrogencarbonat unter langsamen Rühren gelöst. Die Bicarbonatkonzentration wird nach der Formel bestimmt:

Bicarbonatkonzentration [mmol/l] = 5 x Acidität der ersten Einzellösung [mmol/l] x V_{A}/V_{B},
wobei V_{A} das Volumen der ersten Einzellösung und V_{B} das Volumen der zweiten Einzellösung darstellen.

Von dieser berechneten Bicarbonatkonzentration kann um 50 % nach unten und um 100 % nach oben abgewichen werden. Beträgt die Acidität der ersten Einzellösung beispielsweise 0,2 mmol/l und werden gleich große Kompartimente eines Doppelkammerbeutels verwendet, liegt die optimale Bicarbonatkonzentration im Bereich zwischen 0,5 und 2,0 mmol/l.

Die auf diese Weise hergestellten Einzellösungen werden anschließend durch Membransterilfilter in einen Kühltank filtriert. Nach Ansatzkontrolle und Freigabe der Lösung werden diese in einen Doppelkammermehrschichtfolienbeutel gefüllt, wobei die erste Einzellösung in eine erste Kammer und die zweite Einzellösung in eine zweite Kammer gefüllt wird. Beide Kammern sind durch eine Schweißnaht voneinander getrennt. Die Kompartimente werden jeweils mit einem Konnektor verschlossen. Anschließend wird der Doppelkammerbeutel in einen Umbeutel umverpackt und dann bei 121°C Hitze sterilisiert. Nach der Hitzesterilisation wird durch Druck auf eine der Kammern die Schweißnaht zumindest teilweise geöffnet, wonach sich die Lösungen mischen und ein Misch-pH-Wert im Bereich zwischen 6,8 und 7,0, vorzugsweise 6,8 erhalten wird.

## Patentansprüche

1. Verfahren zur Herstellung einer Lösung für die Peritonealdialyse durch Vermischen einer ersten und einer zweiten Einzellösung, wobei die erste und die zweite Einzellösung in einem Doppelkammerbeutel getrennt gelagert sind,
**dadurch gekennzeichnet,**
**dass** die Herstellung der ersten Einzellösung das Lösen eines Glucosepolymers und/oder Glucosepolymer-Derivates und die Einstellung des pH-Wertes im Bereich zwischen 4 und 4,3 umfasst,
**dass** die Herstellung der zweiten Einzellösung das Lösen eines Puffers umfasst, der in einem physiologischen pH Wert von etwa 7 puffert,
**dass** zur ersten Einzellösung ferner Elektrolytsalze zugegeben werden,
**dass** die Einzellösungen hitzesterilisiert werden,
**dass** die erste und die zweite Einzellösung in zwei getrennte Kammern des Doppelkammerbeutels gefüllt werden, die vorzugsweise durch eine Schweißnaht voneinander getrennt sind, und
**dass** Druck auf eine Kammer ausgeübt wird, sodass der Inhalt der beiden Kammern miteinander vermischt wird.

2. Verfahren nach Anspruch 1, **dadurch gekennzeichnet, dass** der pH-Wert der ersten Einzellösung im Bereich zwischen 4,1 und 4,3, und vorzugsweise auf 4,2 eingestellt wird.

3. Verfahren nach einem der vorhergehenden Ansprüche, **dadurch gekennzeichnet, dass** es sich bei dem Glucosepolymer-Derivat um Hydroxyethylstärke handelt.

4. Verfahren nach einem der vorhergehenden Ansprüche, **dadurch gekennzeichnet, dass** die Herstellung der ersten Einzellösung ferner die Zugabe von Calcium-lonen, Natrium-Ionen, Magnesium-Ionen, H+ Überschuss-Ionen und Chlorid-Ionen umfasst.

5. Verfahren nach einem der vorhergehenden Ansprüche, **dadurch gekennzeichnet, dass** der Puffer Bicarbonat enthält.

6. Verfahren nach einem der vorhergehenden Ansprüche, **dadurch gekennzeichnet, dass** die Bicarbonatkonzentration in Abhängigkeit von der Acidität der ersten Einzellösung eingestellt wird und nach der Formel: Bicarbonatkonzentration [mmol/l] = 5 x Acidität der ersten Einzellösung [mmol/l] x V_{A}/V_{B} bestimmt wird, wobei V_{A} das Volumen der ersten Einzellösung und V_{B} das Volumen der zweiten Einzellösung darstellt.

7. Verfahren nach Anspruch 6, **dadurch gekennzeichnet, dass** die Bicarbonatkonzentration in einem Bereich eingestellt wird, dessen Untergrenze durch die Hälfte und dessen Obergrenze durch das Doppelte der nach Anspruch 6 bestimmten Bicarbonatkonzentration gebildet wird.

8. Verfahren nach einem der vorhergehenden Ansprüche, **dadurch gekennzeichnet, dass** der Puffer das Salz einer schwachen Säure, vorzugsweise Lactat, enthält.

9. Verfahren nach einem der vorhergehenden Ansprüche, **dadurch gekennzeichnet, dass** der pH-Wert der ersten Einzellösung durch Zugabe einer physiologisch verträglichen Säure, insbesondere Salzsäure eingestellt wird.

10. Verfahren nach Anspruch 4, **dadurch gekennzeichnet, dass** die Natrium-Ionen, Calcium-Ionen, Magnesium-Ionen, H⁺ Überschuss-Ionen, und Chlorid-Ionen in einer Menge zugegeben werden, dass sie in der ersten Einzellösung in folgenden Konzentrationen vorliegen:
Natrium-Ionen [mmol/l]: 180 - 200
Calcium-Ionen [mmol/l]: 2 - 4
Magnesium-lonen [mmol/l]: 0,8 - 1,2
H⁺ Überschuss-Ionen [mmol/l]: 0,05 - 0,1
Chlorid-Ionen [mmol/l]: 197 - 210

11. Verfahren nach Anspruch 5, **dadurch gekennzeichnet, dass** der zweiten Einzellösung Bicarbonat in einer Menge zugegeben wird, dass es in der zweiten Einzellösung im Konzentrationsbereich von zwischen 0,5 und 2,0 mmol/l, und vorzugsweise in einer Konzentration von 1,0 mmol/l vorliegt.

12. Verfahren nach einem der vorhergehenden Ansprüche, **dadurch gekennzeichnet, dass** die Herstellung der ersten Einzellösung das Lösen von Natriumchlorid, Calciumchlorid, Magnesiumchlorid sowie des Glucosepolymers und/oder des Glucosepolymerderivats und Salzsäure unter Rühren in Wasser umfasst.

13. Verfahren nach einem der vorhergehenden Ansprüche, **dadurch gekennzeichnet, dass** die Herstellung der zweiten Einzellösung durch Lösen von Natriumhydrogencarbonat unter Rühren in Wasser umfasst.

14. Verfahren nach einem der vorhergehenden Ansprüche, ferner umfassen das Filtrieren der Einzellösungen nach deren Herstellung durch einen Membransterilfilter in einen Kühltank.

15. Verfahren nach Anspruch 14, ferner umfassend das Füllen der ersten Einzellösung in eine Kammer und der zweiten Einzellösung in eine andere Kammer des Doppelkammerbeutels.

16. Verfahren nach einem der vorangehenden Ansprüche, **dadurch gekennzeichnet, dass** die Einzellösungen im Doppelkammerbeutel hitzesterilisiert werden.

## Claims

1. A method of producing a solution for peritoneal dialysis by mixing a first and a second individual solution, wherein the first and the second individual solutions are stored separately in a double-chamber bag,
**characterised in that**
production of the first individual solution includes dissolving a glucose polymer and/or a glucose polymer derivative and setting of the pH-value in the range between 4 and 4.3,
production of the second individual solution includes dissolving a buffer which buffers in a physiological pH-value of about 7,
in addition electrolytic salts are added to the first individual solution, the individual solutions are heat-sterilised,
the first and second individual solutions are introduced into two separate chambers of a double-chamber bag, that are preferably separated from each other by a weld seam, and
pressure is applied to a chamber so that the content of the two chambers is mixed together.

2. A method according to claim 1 **characterised in that** the pH-value of the first individual solution is set in the range between 4.1 and 4.3 and preferably to 4.2.

3. A method according to one of the preceding claims **characterised in that** the glucose polymer derivative is hydroxyethylene starch.

4. A method according to one of the preceding claims **characterised in that** production of the first individual solution further includes the addition of calcium ions, sodium ions, magnesium ions, H+ excess ions and chloride ions.

5. A method according to one of the preceding claims **characterised in that** the buffer contains bicarbonate.

6. A method according to one of the preceding claims **characterised in that** the bicarbonate concentration is set in dependence on the acidity of the first individual solution, and is determined according to the formula: bicarbonate concentration [mmol/l]=5 x acidity of the first individual solution [mmol/l] x V_{A}/V_{B}, V_{A} being the volume of the first individual solution and V_{B} being the volume of the second individual solution.

7. A method according to claim 6 **characterised in that** the bicarbonate concentration is set in a range whose lower limit is formed by half of the bicarbonate concentration determined according to claim 6, and whose upper limit is formed by twice the bicarbonate concentration determined according to claim 6.

8. A method according to one of the preceding claims **characterised in that** the buffer contains the salt of a weak acid, preferably lactate.

9. A method according to one of the preceding claims **characterised in that** the pH-value of the first individual solution is set by the addition of a physiologically compatible acid, in particular hydrochloric acid.

10. A method according to claim 4 **characterised in that** the sodium ions, calcium ions, magnesium ions, H+ excess ions and chloride ions are added in an amount such that they are present in the first individual solution in the following concentrations:
sodium ions [mmol/l]: 180-200
calcium ions [mmol/l]: 2-4
magnesium ions [mmol/l]: 0.8-1.2
H+ excess ions [mmol/l]: 0.05-0.1
chloride ions [mmol/l]: 197-210.

11. A method according to claim 5 **characterised in that** bicarbonate is added to the second individual solution in an amount such that it is present in the second individual solution in the concentration range between 0.5 and 2.0 mmol/l, preferably in a concentration of 1.0 mmol/l.

12. A method according to one of the preceding claims **characterised in that** production of the first individual solution includes dissolving sodium chloride, calcium chloride, magnesium chloride and the glucose polymer and/or the glucose polymer derivative and hydrochloric acid with stirring in water.

13. A method according to one of the preceding claims **characterised in that** production of the second individual solution includes dissolving sodium hydrogen carbonate with stirring in water.

14. A method according to one of the preceding claims further including filtering the individual solutions after production thereof by a membrane sterile filter in a cooling tank.

15. A method according to claim 14 further including introducing the first individual solution into one chamber and the second individual solution into another chamber of the double-chamber bag.

16. A method according to one of the preceding claims **characterised in that** the individual solutions are heat-sterilised in the double-chamber bag.

## Revendications

1. Procédé de préparation d'une solution pour la dialyse péritonéale, par mélange d'une première et d'une deuxième solutions individuelles, la première et la deuxième solutions individuelles étant logées séparément dans un sac à deux compartiments,
**caractérisé en ce que**
la préparation de la première solution individuelle comprend la dissolution d'un polymère du glucose et/ou d'un dérivé d'un polymère du glucose, et l'ajustement du pH dans la plage comprise entre 4 et 4,3,
la préparation de la deuxième solution individuelle comprend la dissolution d'un tampon qui tamponne à un pH physiologique d'environ 7,
on ajoute en outre à la première solution individuelle des sels d'électrolytes,
on stérilise à la chaleur les solutions individuelles, la première et la deuxième solutions individuelles sont placées dans deux chambres individuelles du sac à deux compartiments, qui de préférence sont séparées l'une de l'autre par une soudure, et
on exerce sur une pression sur une chambre de façon que les contenus des deux chambres soient mélangés l'un à l'autre.

2. Procédé selon la revendication 1, **caractérisé en ce que** le pH de la première solution individuelle est ajusté à une valeur comprise dans la plage de 4,1 à 4,3, et de préférence à 4,2.

3. Procédé selon l'une des revendications précédentes, **caractérisé en ce que**, pour ce qui concerne le dérivé d'un polymère du glucose, il s'agit d'hydroxyéthyl-amidon.

4. Procédé selon l'une des revendications précédentes, **caractérisé en ce que** la préparation de la première solution individuelle comprend en outre l'addition d'ions calcium, d'ions sodium, d'ions magnésium, d'ions H⁺ en excès et d'ions chlorure.

5. Procédé selon l'une des revendications précédentes, **caractérisé en ce que** le tampon contient du bicarbonate.

6. Procédé selon l'une des revendications précédentes, **caractérisé en ce que** la concentration du bicarbonate est ajustée en fonction de l'acidité de la première solution individuelle et est déterminée selon la formule : concentration du bicarbonate [mmol/l] = 5 x acidité de la première solution individuelle [mmol/l] x V_{A}/V_{B}, où V_{A} est le volume de la première solution individuelle et V_{B} le volume de la deuxième solution individuelle.

7. Procédé selon la revendication 6, **caractérisé en ce que** la concentration du bicarbonate est ajustée dans une plage dont la limite inférieure est formée par la moitié et dont la limite supérieure est formée par le double de la concentration du bicarbonate déterminée dans la revendication 6.

8. Procédé selon l'une des revendications précédentes, **caractérisé en ce que** le tampon contient le sel d'un acide faible, de préférence un lactate.

9. Procédé selon l'une des revendications précédentes, **caractérisé en ce que** le pH de la première solution individuelle est ajusté par addition d'un acide physiologiquement acceptable, en particulier de l'acide chlorhydrique.

10. Procédé selon la revendication 4, **caractérisé en ce que** les ions sodium, les ions calcium, les ions magnésium, les ions H⁺ en excès et les ions chlorure sont ajoutés en une quantité telle qu'ils se retrouvent dans la première solution individuelle en les concentrations suivantes :
ions sodium [mmol/l] : 180-200
ions calcium [mmol/l] : 2-4
ions magnésium [mmol/l] : 0,8-1,2
ions H⁺ en excès [mmol/l] : 0,05-0,1
ions chlorure [mmol/l] : 197-210.

11. Procédé selon la revendication 5, **caractérisé en ce que** du bicarbonate est ajouté à la deuxième solution individuelle en une quantité telle qu'il se retrouve dans la deuxième solution individuelle dans une plage de concentrations comprise entre 0,5 et 2,0 mmol/l, et de préférence à une concentration de 1,0 mmol/l.

12. Procédé selon l'une des revendications précédentes, **caractérisé en ce que** la préparation de la première solution comprend la dissolution, sous agitation dans l'eau, de chlorure de sodium, de chlorure de calcium, de chlorure de magnésium, ainsi que du polymère du glucose et/ou du dérivé d'un polymère du glucose et d'acide chlorhydrique.

13. Procédé selon l'une des revendications précédentes, **caractérisé en ce que** la préparation de la deuxième solution individuelle a :lieu par dissolution d'hydrogénocarbonate de sodium sous agitation dans l'eau.

14. Procédé selon l'une des revendications précédentes, comprenant en outre la filtration, à travers une membrane filtrante stérile dans une cuve de refroidissement, des solutions individuelles après leur préparation.

15. Procédé selon la revendication 14, comprenant en outre la mise en place de la première solution individuelle dans une chambre et celle de la deuxième solution individuelle dans une autre chambre du sac à deux compartiments.

16. Procédé selon l'une des revendications précédentes, **caractérisé en ce que** les solutions individuelles sont stérilisées à la chaleur dans le sac à deux compartiments.
